(19) **Europäisches Patentamt / European Patent Office / Office européen des brevets**

(11) **EP 1 657 548 B1**

(12) **EUROPEAN PATENT SPECIFICATION**

(45) Date of publication and mention
of the grant of the patent:
**21.07.2010 Bulletin 2010/29**

(21) Application number: **04762075.2**

(22) Date of filing: **12.08.2004**

(51) Int Cl.:
*G01N 33/48* (2006.01)   *B01D 59/28* (2006.01)
*C01B 4/00* (2006.01)

(86) International application number:
**PCT/CN2004/000939**

(87) International publication number:
**WO 2005/050196 (02.06.2005 Gazette 2005/22)**

(54) **METHOD OF CONFIRMING HYDROGEN/DEUTERIUM EXCHANGE IN CONSTRUCTIONS OF BIOLOGICAL SAMPLES AND DETERMINING THE PERCENTAGE OF DEUTERIUM IN BIOLOGICAL SAMPLES**

VERFAHREN ZUR BESTÄTIGUNG DES WASSERSTOFF/DEUTERIUM-AUSTAUSCHS IN KONSTRUKTIONEN BIOLOGISCHER PROBEN UND ZUR BESTIMMUNG DES PROZENTANTEILS VON DEUTERIUM IN BIOLOGISCHEN PROBEN

PROCEDE DE CONFIRMATION DE L'ECHANGE HYDROGENE/DEUTERIUM DANS DES CONSTRUCTIONS D'ECHANTILLONS BIOLOGIQUES ET DETERMINATION DU POURCENTAGE DE DEUTERIUM DANS DES ECHANTILLONS BIOLOGIQUES

(84) Designated Contracting States:
**AT BE BG CH CY CZ DE DK EE ES FI FR GB GR HU IE IT LI LU MC NL PL PT RO SE SI SK TR**

(30) Priority: **13.08.2003   CN 03142290**

(43) Date of publication of application:
**17.05.2006   Bulletin 2006/20**

(73) Proprietors:
- **Zhejiang Pukang Biotechnology Co., Ltd.**
  **Hangzhou,**
  **Zhejiang 310013 (CN)**
- **Zhejiang University**
  **Hangzhou, Zhejiang 310027 (CN)**
- **Zhejiang Academy Of Medical Sciences**
  **Hangzhou,**
  **Zhejiang 310013 (CN)**

(72) Inventors:
- **MAO, Jiangsen**
  **Hangzhou,**
  **Zhejiang 310013 (CN)**
- **LIU, Ziyang**
  **Hangzhou,**
  **Zhejiang 310013 (CN)**
- **TANG, Caihua**
  **Hangzhou,**
  **Zhejiang 310013 (CN)**
- **HE, Yihui**
  **Hangzhou,**
  **Zhejiang 310013 (CN)**
- **ZHU, Jiahong**
  **Hangzhou,**
  **Zhejiang 310013 (CN)**
- **CHEN, Yueqing**
  **Hangzhou,**
  **Zhejiang 310013 (CN)**
- **MAO, Zixu**
  **Hangzhou,**
  **Zhejiang 310013 (CN)**
- **CHAI, Shaoai**
  **Hangzhou,**
  **Zhejiang 310013 (CN)**

(74) Representative: **Kupecz, Arpad**
  **Octrooibureau Los en Stigter B.V.**
  **P.O. Box 20052**
  **1000 HB Amsterdam (NL)**

(56) References cited:
**US-A- 5 272 054**

- **WONG W W ET AL: "DEUTERIUM AND OXYGEN-18 MEASUREMENTS ON MICROLITER SAMPLES OF URINE PLASMA SALIVA AND HUMAN MILK" AMERICAN JOURNAL OF CLINICAL NUTRITION, vol. 45, no. 5, 1987, pages 905-913, XP002446377 ISSN: 0002-9165**

- WONG W W ET AL: "MEASUREMENT OF IN-VIVO CHOLESTEROL SYNTHESIS FROM HEAVY WATER A RAPID PROCEDURE FOR THE ISOLATION COMBUSTION AND ISOTOPIC ASSAY OF ERYTHROCYTE CHOLESTEROL" JOURNAL OF LIPID RESEARCH, vol. 32, no. 6, 1991, pages 1049-1056, XP002446378 ISSN: 0022-2275
- YIHUI HE ET AL: "A method for quantitative determination of deuterium content in biological material" RAPID COMMUNICATIONS IN MASS SPECTROMETRY WILEY UK, vol. 19, no. 6, 2005, pages 838-842, XP002446379 ISSN: 0951-4198
- MAO J. ET AL.: 'Confirmation of deuterium/ hydrogen exchange in virus particles' SCIENCE LETTER vol. 49, no. 1, January 2004, pages 95 - 98, XP008082262
- COLEMAN M.L. ET AL.: 'Reduction of water with zinc for hydrogen isotope analysis' ANAL. CHEM. vol. 54, no. 6, 1982, pages 993 - 995, XP003017543
- WU R. ET AL.: 'Thermostabilization of live virus vaccines by heavy water (D2O)' VACCINE vol. 13, no. 12, 1995, pages 1058 - 1063, XP004057493
- TANWEER A. ET AL.: 'Importnace of clean metallic zinc for hydrogen isotope analysis' ANAL. CHEM. vol. 62, no. 19, 01 October 1990, pages 2158 - 2160, XP003017544
- XU WEI ET AL.: 'Measurement of hydrogen-deuterium ratio and particle recycling' NUCLEAR FUSION AND PLASMA PHYSICS vol. 21, no. 2, June 2001, pages 119 - 123, XP008082266
- JIANGSEN M. ET AL CHINESE SCIENCE BULLETIN vol. 49, no. 3, February 2004, pages 253 - 257

**Description**

TECHNICAL FIELD

The invention relates to a method for analyzing hydrogen isotopes, especially a method for determining the percent content of deuterium in the biological sample.

BACKGROUND ART

Since Urey *et al.* found deuterium, one of the hydrogen isotopes, in 1932, deuterium has been widely used in the biomedical field. The effect of heavy water on organisms, such as the effect of heavy water on the growth and development of bacteria, fungi, plants, mammal and the like, has been widely studied. In 1965, Mao Jiangsen, Huang Zhenxiang *et al.* found that Japanese encephalitis virus (JEV) which is extremely sensitive to heat has an increased heat stability when reproducing in cells treated with heavy water (Mao CS, Huang CH. Sci. Sin. 1965, 14(6): 885~890). In recent years, studies showed that poliomyelitis virus, influenza virus and hepatitis A virus (HAV) have a similar result when treated with heavy water (Wu R, Georgescu MM, Delpeyroux F. Vaccine, 1995, 13:1058~1063; and Ikizler MR, Wright PF. Vaccine, 2002, 20:1393~1399). However, the real mechanism about the effect of heavy water on these viruses is still unclear. Although exchange of hydrogen for deuterium in viral structures may be the rational explanation, hitherto, there are no direct methods capable of confirming hydrogen/deuterium exchange in viral particles and determining deuterium content thereof. Thus, deuteration conditions of biological samples cannot be optimized, and processes of deuteration cannot be normalized.

Studies about the analysis methods of hydrogen isotopes have been carried out for a long time, and hitherto, there are many analysis methods, such as cryoscopic method, refractive index method, resistivity method, thermal electric conductivity method, infra-red spectrometry, neutron thermal energy spectrometry, gas chromatography, nuclear magnetic resonance (NMR) and chemical ionization mass spectrometry and the like (Abrams, S. A., Wong, W. W. (Book) New York CABI Publishing, 2003; and Platzner IT, Habfast K, Walder AJ. et al. (Book) New York John Wiley & Sons Ltd., 1997). Stable isotope deuterium tracer mass spectrometry is an analysis method that is widely used in the area of biomedical research, and is extensively used for the determination and identification of metabolic products, the studies about the mechanism of metabolic pathway, and the determination of the concentration of medicaments and metabolic products in organisms. In this method, man can determine the molecular composition of the substance to be tested and thereby know the property of the substance to be tested, as well as determine the content of the substance to be tested, by measuring the intensity of molecular ions or few characteristic fragment ions. This method does not require an intact mass spectrogram, i.e., it is only required to separate the interested compound, and then obtain a part of mass spectrogram using ion monitoring technique and determine the ratio between labeled and unlabeled components. The accuracy of the ratio determined by this method is very low, and generally 1~10%, whereas the method has specificity and high sensitivity. Separation techniques that were developed in the recent twenty years, such as capillary separation technique, greatly improve the accuracy, sensitivity, selectivity and exclusivity of stable isotope deuterium tracer mass spectrometry, and thus gradually become the most important analysis methods in the area of biomedical research. However, these methods also have some limitations: (1) the test instruments used in these methods are mainly gas chromatograph-mass spectrometer (GC-MS) and liquid chromatograph-mass spectrometer (LC-MS), which are expensive; (2) highly pure medicaments labeled with stable isotope deuterium are obtained hardly, their amount is small and the cost of synthesis is high; and (3) the treatment of sample and the manipulation of testing and analyzing techniques are complex and time consuming.

$^2$H-NMR method is widely used in biochemistry and clinical medicine due to its advantages such as going deep into the interior of substance, not destroying the sample, and providing parameters, e.g. the molecular weight and structure of biological molecules (Armellin S, Brenna E, Fronza G, et al. Analyst. 2004 Feb, 129(2):130-3; and Roger O, Lavigne R, Mahmoud M, et al. J Biol Chem. 2004 Jun 11, 279(24):24923-8). The abundance of deuterium in nature is as low as about $150\times10^{-6}$, and its sensitivity is about $150\times10^{-6}$ of that of hydrogen. The nuclear magnetic moment of deuteron is smaller, its spin quantum number is 1, the gyro-magnetic ratio of deuteron is lower, and thus the resonance frequency of deuteron and the dispersity of spectral line are low. Therefore, the sensitivity of $^2$H-NMR method is very low. Accordingly, in order to achieve satisfactorily a signal-to-noise ratio and an accuracy of determination, larger amount of sample and longer analysis time are required, and it is needed to purify the sample prior to determination.

In recent years, the rapidly developing life science raised a need to determine the content of deuterium in a biological sample. In some areas of life science, such as the research on JEV and HAV, in general, deuterium-rich biological samples can only be provided at a level of microgram. If it is required to confirm whether hydrogen/deuterium exchange has occurred in the whole viral particle and to determine the percent content of deuterium in the viral particle, all the existing analysis method for hydrogen isotopes cannot be directly used for the analysis of hydrogen isotopes in these biological samples.

WONG W W ET AL: AMERICAN JOURNAL OF CLINICAL NUTRITION, vol. 45, no. 5, 1987, pages 905-913, XP002446377 ISSN: 0002-9165 discloses deuterium and oxygen-18 measurements on microliter samples of urine, plasma, saliva, and human milk .

WONG W W ET AL: JOURNAL OF LIPID RESEARCH, vol. 32, no. 6, 1991, pages 1049-1056 discloses measurement of in vivo cholesterol synthesis from $^2H_2O$: a rapid procedure for the isolation, combustion, and isotopic assay or erythrocyte cholesterol.

MAO J. ET AL.: CHINESE SCIENCE BULLETIN vol. 49, no. 3, February 2004, pages 253-257 relates to evidence for hydrogen/deuterium exchange in viral particles.

## CONTENTS OF THE INVENTION

The aim of present invention is to provide a novel analysis method for hydrogen isotopes in order to demonstrate whether hydrogen/deuterium exchange has occurred in a biological sample of interest and to calculate the percent content of deuterium in the biological sample. Further studies can ascertain the optimal percent content of deuterium in biological samples and the optimal deuteration conditions of biological samples, and thus improve the stability of biological samples, such as polypeptide. The percent content of deuterium in a deuterated biological sample is determined by the method defined in the claims, and the result shows that its percent content of deuterium is increased. By other experiments, it is proved that the biological characters of this deuterated biological sample are markedly changed, and mainly, the heat resistance is markedly changed.

The present invention provides a method for determining the percent content of deuterium in the structure of a biological sample, comprising the following steps:

(1) selecting a known natural protein as matrix, and formulating it with PBS to form a matrix solution having an appropriate concentration;

(2) diluting a biological sample obtained after purification with PBS to various concentrations, subjecting multiple sample solutions that have different concentrations but the same volume respectively to the following manipulations: mixing homogeneously the sample solution with a suitable amount of the matrix solution followed by lyophilization to form a lyophilized mixed sample, sufficiently oxidizing the lyophilized mixed sample with oxidants through burning in order to oxidize the hydrogen in the mixed sample to water, reacting the resulting water after separation with zinc to generate hydrogen gas, and determining the $^2H/^1H$ ratio of the generated hydrogen gas with a gas isotope mass spectrometer; and

(3) according to a formula, calculating the percent content of deuterium in the biological sample by utilizing the above $^2H/^1H$ ratios of hydrogen gas determined for the multiple samples.

In one embodiment of the above-mentioned determination method, said step (3) is carried out as follows:

(a) plotting the determined $^2H/^1H$ ratios of the multiple samples (expressed with $\delta D_{SA-SMOW}$) against the amount of biological sample/amount of matrix protein (expressed with $m_{bio}/m_{MAT}$), and thus obtaining a straight line having a good linear correlation, $\delta D_{SA-SMOW} = k \cdot \dfrac{m_{bio}}{m_{MAT}} + \delta D_{MAT-SMOW}$, and educing the slope of the straight line,

$$k = \frac{500 \cdot C_{bio}^{D}}{R_{SMOW} \cdot C_{MAT}^{H}};$$

(b) calculating the theoretical percent content of $^1H$ in the matrix protein, $C_{MAT}^{H}$, according to the amino acid composition of the matrix protein;

(c) according to the slope k, calculating the percent content of deuterium in the biological sample as

$$C_{bio}^{D} = k \times R_{SMOW} \times C_{MAT}^{H} \times \frac{1}{500},$$ wherein $R_{SMOW}$ represents the $^2H/^1H$ ratio of SMOW and its value is

$155.76 \times 10^{-6}$.

In the determination method according to the present invention, said known natural protein is bovine serum albumin (BSA).

In one embodiment of the determination method according to the present invention, said biological sample is a microorganism sample or a biomacromolecule sample. In its preferred embodiments, said biomacromolecule is a ribonucleic acid (RNA), a protein or a polypeptide.

In a further aspect, the present invention provides use of the above-mentioned determination method in ascertaining the deuterium content of a biological sample having the optimal heat stability or ascertaining the optimal deuteration conditions of a biological sample.

In a further aspect, the present invention provides use of the above-mentioned determination method in confirming the close correlation between the stability and heat resistance of a microorganism or a biomacromolecule and the deuterium content thereof.

A determination method according to the present invention comprises the following steps: selecting a matrix, mixing homogeneously a prepared biological sample with a suitable amount of the matrix followed by lyophilization to form a lyophilized mixed sample, sufficiently oxidizing the lyophilized mixed sample with oxidants through burning in order to oxidize the hydrogen in the mixed sample to water, reacting the resulting water after separation with zinc to generate hydrogen gas. The $^2H/^1H$ ratio of the generated hydrogen gas is determined with a gas isotope mass spectrometer, wherein the $^2H/^1H$ ratio of the sample is expressed with $\delta D_{SA-SMOW}$, i.e. the change value of deuterium/hydrogen ratio of the sample relative to deuterium/hydrogen ratio of the international standard - Standard Mean Ocean Water (SMOW). Comparison between the results determined from samples prepared from a deuterated biological sample and those of a corresponding natural biological sample is made so as to confirm whether hydrogen/deuterium exchange has occurred in the microorganism and biomacromolecule, and the percent content of deuterium in the biological sample is calculated using corresponding formula. Further studies can ascertain the optimal deuterium content of a biological sample or the optimal deuteration conditions, and confirm that the stability and heat resistance of a microorganism or biological molecule are closely correlative with the deuterium content thereof.

A specific method according to the present invention comprises selecting a known natural protein as matrix and subjecting each of purified biological samples to the following manipulations: diluting the sample with phosphate-buffered saline (PBS) to form a sample solution having a desired concentration, taking 0.1 ml of the sample solution and adding it to 0.5 ml of a matrix solution containing 5 mg known natural protein to form a mixed sample, placing then the mixed sample into a lyophil apparatus of Type GEL-1 for lyophilization to such an extent that the water content of the mixed sample is <3% after lyophilization (determined by Karl Fischer Titrator method), removing adsorbed water in the lyophilized mixed sample and CuO (500 mg) and $V_2O_5$ (50 mg) as oxidants at a temperature of 100°C for 15-30 minutes, and oxidizing the lyophilized mixed sample with the oxidants by burning at 800-950°C for 15-30 minutes so that the sample is fully oxidized to $CO_2$, $H_2O$, $NO_2$ and so on after complete reaction, separating the water in the product and transferred it into a reaction tube for water sample, and then reacting the water with zinc at 400-450°C for 1.5-2.5 hours and thereby converting into hydrogen gas, determining the $^2H/^1H$ ratio of the hydrogen gas with a gas isotope mass spectrometer MAT-251.

The $\delta D_{SA-SMOW}$ value of the hydrogen isotope in sample is represented with the change of the deuterium/hydrogen ratio of sample relative to the deuterium/hydrogen ratio of SMOW, and is defined as follows:

$$\delta D_{SA-SMOW} = \frac{R_{SA} - R_{SMOW}}{R_{SMOW}} \times 10^3$$

wherein $R_{SA}$ represents the $^2H/^1H$ ratio of sample; $R_{SMOW}$ represents the $^2H/^1H$ ratio of SMOW (which value is $155.76 \times 10^{-6}$); only the integral value of $\delta D_{SA-SMOW}$ is generally adopted. From the $\delta D_{SA-SMOW}$ value of sample, the percent content of deuterium in the biological sample can be calculated.

A specific determination method according to the present invention comprises the following steps:

1. selecting a known natural protein as matrix, and formulated it with PBS to form a matrix solution having a concentration of 10 mg/ml.

2. after purification, diluting each of biological samples with PBS to form a diluted sample solution having a desired concentration.

3. taking 0.1 ml of the diluted solution of biological sample and added it into 0.5 ml of the matrix solution having a concentration of 10 mg/ml, and then placing the mixed sample into a lyophil apparatus of Type GEL-1 for lyophilization.

4. removing adsorbed water in the lyophilized mixed sample and the oxidants CuO (500 mg) and $V_2O_5$ (50 mg) at a temperature of 100°C for 15-30 minutes, and then oxidizing the lyophilized mixed sample with the oxidants by burning at 800-950°C for 15-30 minutes so that after complete reaction the sample is fully oxidized to $CO_2$, $H_2O$, $NO_2$ and so on, separating the water in the product and transferred it into a reaction tube for water sample, and then reacting the water with zinc at 400 ˚C-450˚C for 1.5-2.5 hours and thereby converting into hydrogen gas, determining the $^2$H/$^1$H ratio of hydrogen gas with a gas isotope mass spectrometer MAT-251.

5. According to the $\delta D_{SA-SMOW}$ of sample, calculating the percent content of deuterium in the biological sample, wherein the specific calculation formulae are described as follows.

Step I,

$$\delta D_{SA-SMOW} = \frac{R_{SA} - R_{SMOW}}{R_{SMOW}} \times 10^3 \qquad (1)$$

wherein $R_{SA}$ represents the $^2$H/$^1$H ratio of mixed sample, $R_{SMOW}$ represents the $^2$H/$^1$H ratio of SMOW and its value is $155.76 \times 10^{-6}$.

Step II, $R_{SA}$ represents the $^2$H/$^1$H ratio of mixed sample consisting of matrix protein and biological sample, wherein each of the parameters is showed in Table 1.

Table 1. The parameters of matrix protein and biological sample

| Substance | Symbol | Amount Of substance ($\mu$g) | Percent Content of Hydrogen (%) | Percent Content of Deuterium (%) |
|---|---|---|---|---|
| matrix protein | MAT | $m_{MAT}$ | $C_{MAT}^{H}$ | $C_{MAT}^{D}$ |
| biological sample | bio | $m_{bio}$ | $C_{bio}^{H}$ | $C_{bio}^{D}$ |

$$R_{SA} = \frac{\frac{1}{2}\left(m_{MAT} \cdot C_{MAT}^{D} + m_{bio} \cdot C_{bio}^{D}\right)}{m_{MAT} \cdot C_{MAT}^{H} + m_{bio} \cdot C_{bio}^{H}} \approx \frac{1}{2} \cdot \frac{C_{bio}^{D}}{C_{MAT}^{H}} \cdot \frac{m_{bio}}{m_{MAT}} + \frac{1}{2} \cdot \frac{m_{MAT} \cdot C_{MAT}^{D}}{m_{MAT} \cdot C_{MAT}^{H}}$$

$$= \frac{1}{2} \cdot \frac{C_{bio}^{D}}{C_{MAT}^{H}} \cdot \frac{m_{bio}}{m_{MAT}} + R_{MAT} \cdots \left(\because m_{MAT} \cdot C_{MAT}^{H} >> m_{bio} \cdot C_{bio}^{H}\right) \cdots (2)$$

Step III,

$$\delta D_{SA-SMOW} = \frac{R_{SA} - R_{SMOW}}{R_{SMOW}} \times 10^3 = \frac{\frac{1}{2} \cdot \frac{C_{bio}^{D}}{C_{MAT}^{H}} \cdot \frac{m_{bio}}{m_{MAT}} + R_{MAT} - R_{SMOW}}{R_{SMOW}} \times 10^3$$

$$= \frac{500 \cdot C_{bio}^{D}}{R_{SMOW} \cdot C_{MAT}^{H}} \cdot \frac{m_{bio}}{m_{MAT}} + \delta D_{MAT-SMOW} \qquad (3)$$

wherein $R_{SMOW}$ is $155.76 \times 10^{-6}$, $\delta D_{MAT-SMOW}$ can be determined, $C_{MAT}^{H}$ can be calculated via its amino acid composition, and $C_{bio}^{D}$ of biological sample is an unknown constant.

Step IV, if $k$ is defined as $k = \dfrac{500 \cdot C_{bio}^{D}}{R_{SMOW} \cdot C_{MAT}^{H}}$, Formula (3) can be transformed to

$$\delta D_{SA-SMOW} = k \cdot \frac{m_{bio}}{m_{MAT}} + \delta D_{MAT-SMOW} \qquad (4)$$

and thereby there is a linear correlation between $\delta D_{SA-SMOW}$ and $\dfrac{m_{bio}}{m_{MAT}}$.

The gas isotope mass spectrometry assisted by matrix has obvious advantages that: firstly, the result of determination is precise and has a good repeatability; secondly, the amount of sample used in this method is extremely small; thirdly, the determination method is simple and easy to manipulate, and has low cost; fourthly, there is a linear correlation between the value of $\delta D_{SA-SMOW}$ of sample and the amount of biological sample added, and thus the value of $C_{bio}^{D}$, i.e. the percent content of deuterium in biological sample, can be calculated from the slope $k$ of straight line or directly via Formula (3).

Description of Figures

Fig. 1 shows the results of comparison experiments about the heat stability of JEVs cultured in different environments. The deuterated JEV (cultured with 36% $D_2O$) and the non-deuterated JEV (cultured without $D_2O$) are inactivated by heat at 50°C, and the decrease in Plaque Forming Unit (PFU) titer is plotted against time. When the inactivation times are the same, the decrease in PFU titer of the deuterated JEV is smaller than that of the non-deuterated JEV. In Fig. 1, $V_D$ represents the deuterated JEV, and $V_H$ represents the non-deuterated JEV.

Fig. 2 shows the results of comparison experiments about the stability of HAVs cultured in different environments. The deuterated HAV (HAV particle resuspended in 87% $D_2O$) and the non-deuterated HAV (HAV particle resuspended in $H_2O$) are stayed at 10°C, and the decrease in Medial Cell Culture Infectious Dose ($CCID_{50}$) titer is plotted against time. When the standing times are the same, the decrease in $CCID_{50}$ titer of the deuterated HAV is smaller than that of the non-deuterated HAV. In Fig. 2, $V_D$ represents the deuterated HAV, and $V_H$ represents the non-deuterated HAV.

Fig. 3 shows the $\delta D_{SA-SMOW}$ - $m_{bio}/m_{BSA}$ graph of $V_D$ sample prepared from the deuterated HAV.

Fig. 4 shows the $\delta D_{SA-SMOW}$ - $m_{bio}/m_{BSA}$ graph of RNA-$V_D$ sample prepared from ribonucleic acid (RNA) of the deuterated HAV.

Fig. 5 shows the $\delta D_{SA-SMOW}$ - $m_{bio}/m_{BSA}$ graph of the deuterated bovine serum albumin (BSA) sample.

MODE OF CARRYING OUT THE INVENTION

The following non-limiting examples are intended to explain the present invention in more detail, but not to limit the scope of the present invention in any way.

Example 1

Confirmation of hydrogen/deuterium exchange in the whole viral particle of JEV

The primary chick embryo cells (CECs) were used to reproduce JEV. Firstly, CECs were allowed to grow in a culture medium without heavy water ($D_2O$) for 24h, and then were cultured in a maintenance medium containing 36% $D_2O$ at 37°C for 2 days. The maintenance medium containing heavy water was discarded, and JEV was added into the cells and adsorbed for 2h. After adsorption, the adsorption solution was discarded, the cells were washed twice with PBS, and then a fresh maintenance medium containing $D_2O$ was added, and the resulting deuterated JEV (represented with $V_D$) was harvested after 3 days. In above-mentioned culture process, when CECs were cultured with a culture medium without $D_2O$ and infected with JEV, a non-deuterated JEV (represented with $V_H$) was obtained. In above-mentioned culture process, when CECs were cultured with a culture medium containing $D_2O$ but were not infected with JEV, a deuterated cell control without virus (represented with $C_D$) was obtained. All samples of virus and the corresponding cell control without virus were purified by precipitation with polyethylene glycol (PEG), filtration with 0.45 $\mu$m filter, ultracentrifugation with 40% sucrose underlaid, and the like. The virus samples were inactivated with formaldehyde, and then their titers were determined by hemagglutination method and expressed with Hemagglutination Unit (HAU).

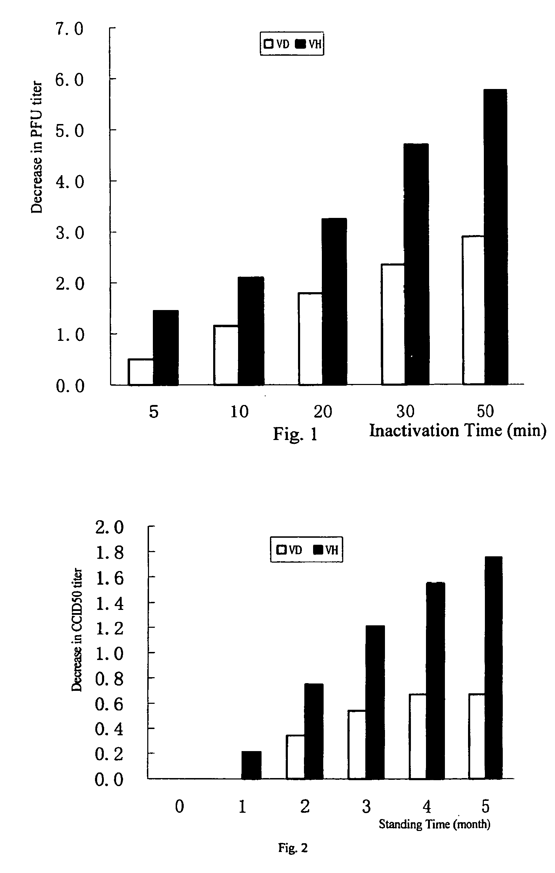

It was found that the decrease in PFU titer of the deuterated JEV and that of the non-deuterated JEV varied with the inactivation time at 50˚C, and the decrease in PFU titer of the non-deuterated JEV was greater than that of the deuterated JEV under the same inactivation time (see Fig. 1). This indicated that JEV reproduced in the presence of $D_2O$ had an increased heat resistance.

BSA was selected as matrix. After purification, the deuterated JEV, the non-deuterated JEV and the deuterated cell control without virus were subjected respectively to the following manipulations: it was diluted with PBS to form a diluted sample solution having a desired concentration, 0.1 ml of the diluted sample solution was taken and added into 0.5 ml of a PBS solution containing 5 mg BSA. Then, the resulting mixed sample was placed into a lyophil apparatus of Type GEL-1 for lyophilization to the extent that the water content of the mixed sample was <3% after lyophilization. The resulting samples were represented with $V_D$, $V_H$ and $C_D$, respectively. Adsorbed water in the lyophilized mixed sample and the oxidants CuO (500 mg) and $V_2O_5$ (50 mg) was removed at a temperature of 100˚C for 20 minutes, and then the oxidation reaction of the lyophilized mixed sample with the oxidants was carried out by burning at 850˚C for 30 minutes so that after complete reaction the sample was fully oxidized to carbon dioxide ($CO_2$), water ($H_2O$), nitrogen dioxide ($NO_2$) and so on. The water in the product was separated and transferred into a reaction tube for water sample, and then the water was reacted with zinc at 420˚C for 2 hours and thereby converted into hydrogen gas, the $^2H/^1H$ ratio of which was determined with a gas isotope mass spectrometer MAT-251. The determined values of $\delta D_{SA-SMOW}$ of samples $V_D$, $V_H$ and $C_D$ were shown in Table 2.

Table 2. The values of $\delta D_{SA-SMOW}$ of samples $V_D$ $V_H$ and $C_D$

| Sample | $\delta D_{SA-SMOW}$ | | | |
|---|---|---|---|---|
| | Sample ($\mu$g) | | | |
| | 0 | 5 | 10 | 20 |
| $V_D$ | | -35$\pm$3 | -8$\pm$4 | 42$\pm$3 |
| $V_H$ | -76$\pm$3 | -72$\pm$2 | -54$\pm$0 | -60$\pm$0 |
| $C_D$ | | -75$\pm$3 | -65$\pm$0 | -70$\pm$7 |

Because the value of $\delta D_{SA-SMOW}$ of the matrix BSA was a constant value, the values of $\delta D_{SA-SMOW}$ of the mixed samples reflected the levels of $^2H/^1H$ ratio of the added biological samples, and reflected the added amounts thereof. When the added amounts were the same, a larger value of $\delta D_{SA-SMOW}$ of the mixed sample indicated a higher $^2H/^1H$ ratio of the biological sample; when the added biological samples were from the same source, a larger value of $\delta D_{SA-SMOW}$ of the mixed sample indicated a larger added amount of the biological sample.

In Table 2, it can be seen that the value of $\delta D_{SA-SMOW}$ of sample $V_D$ was larger than that of sample $V_H$ when the added amounts of JEV were the same; and the value of $\delta D_{SA-SMOW}$ of sample $V_D$ increased with the increase in the added amount of virus. The increase in $\delta D_{SA-SMOW}$ of sample $V_D$ was unlikely to be obtained from the possible contaminating cellular components during the preparation of viral sample, because the values of $\delta D_{SA-SMOW}$ of sample $C_D$, the cellular components control without virus, were similar at different dosages, and ranged from -65 to -75, there was no positive correlation with the dosage, and the $\delta D_{SA-SMOW}$ of sample $C_D$ was relatively close to that of the matrix BSA. This indicated that the deuterium in sample $C_D$, the cellular components control, was under the natural state. Therefore, compared with JEV obtained in the culture medium without $D_2O$, the $^2H/^1H$ ratio of the JEV reproduced in the presence of $D_2O$ was increased significantly, i.e., the $^2H/^1H$ ratio of the deuterated JEV was higher than that of the non-deuterated JEV. This indicated that deuterium was enriched in the JEV reproduced in the presence of $D_2O$, i.e. the deuterium content thereof was increased, and that deuterium atoms replaced some hydrogen atoms in the JEV particle.

Example 2

Confirmation of hydrogen/deuterium exchange in whole viral particle of HAV and determination of the percent content of deuterium thereof

The vaccine strain of hepatitis A virus (Strain H2) was reproduced in and obtained from the diploid cells of human lung. The cells infected with Strain H2 and the non-infected cells, the control, were harvested, and then resuspended with PBS, and were treated three times with freeze-thaw and ultrasonication, and then centrifugated at 8000 g for 30 minutes to remove the cell debris. The obtained supernatant was extracted three times with chloroform, and then the virus or the cell control was harvested by ultracentrifugation. The deuteration of sample was carried out as follows: the sample was resuspended in a minimum essential medium (MEM) containing 87% heavy water, and incubated at 36°C for one week. The deuterated sample was subjected to ultracentrifugation, the MEM containing heavy water was discarded, and the precipitate was washed well with distilled water and then ultracentrifuged again to harvest the deuterated sample. The washing process was repeated three times to ensure that the deuterated sample almost did not contain free heavy water. The $CCID_{50}$ titer of viral sample was determined by a cell culture method.

It was found that the decrease in $CCID_{50}$ titer of the deuterated HAV and that of the non-deuterated HAV varied with the standing time at 10°C, and the decrease in $CCID_{50}$ titer of the non-deuterated HAV was greater than that of the deuterated HAV under the same standing time (see Fig. 2). This indicated that HAV incubated in the presence of $D_2O$ had an increased stability.

BSA was selected as matrix. The deuterated HAV, the non-deuterated HAV and the deuterated cell control were subjected respectively to the following manipulations: it was diluted with PBS to form a diluted sample solution having a desired concentration, 0.1 ml of the diluted sample solution was taken and added into 0.5 ml of PBS solution containing 5 mg BSA. Then, the mixed sample was placed into a lyophil apparatus of Type GEL-1 for lyophilization to such an extent that the water content of the mixed sample was <3% after lyophilization. The resulting samples were represented with $V_D$, $V_H$ and $C_D$, respectively. Adsorbed water in the lyophilized mixed sample and the oxidants CuO (500 mg) and $V_2O_5$ (50 mg) was removed at a temperature of 100°C for 20 minutes, and then the oxidation reaction of the lyophilized mixed sample with the oxidants was carried out by burning at 850°C for 30 minutes so that after complete reaction the sample was fully oxidized to carbon dioxide ($CO_2$), water ($H_2O$), nitrogen dioxide ($NO_2$) and so on. The water in the product was separated and transferred into a reaction tube for water sample, and then the water was reacted with zinc at 420°C for 2 hours and thereby converted into hydrogen gas, the $^2H/^1H$ ratio of which was determined with a gas isotope mass spectrometer MAT-251.

The $\delta D_{SA-SMOW}$ values of sample $V_D$ prepared from the deuterated HAV were determined. The significant increase in $\delta D_{SA-SMOW}$ value of sample $V_D$ cannot be resulted from the free water in the deuterated HAV particle, because the $\delta D_{SA-SMOW}$ value of the last wash solution (SV) was consistent with that of distilled water; and this cannot be resulted from the possible contaminating deuterated cellular components during the preparation of viral sample neither, because the $\Delta D_{SA-SMOW}$ value of sample $C_D$ was at the background level. Therefore, the significant increase in $\delta D_{SA-SMOW}$ value of sample $V_D$ indicated that the $^2H/^1H$ ratio of HAV was increased after incubation in $D_2O$, and the hydrogen/deuterium exchange occurred in the HAV particles and deuterium was enriched in them. Furthermore, the $\delta D_{SA-SMOW}$ value of sample $V_D$ increased linearly with the increase in the added amount of deuterated HAV, i.e., the $\delta D_{SA-SMOW}$ value of sample $V_D$ was directly proportional to the added amount of deuterated HAV within a defined range. The relation between the $\delta D_{SA-SMOW}$ value of sample $V_D$ and the added amount of deuterated HAV was shown in Table 3.

Table 3. The relation between the $\delta D_{SA-SMOW}$ of sample $V_D$ prepared from deuterated HAV and the added amount of deuterated HAV

| Amount of the deuterated HAV ($m_{bio}$, μg) | Amount of the matrix BSA ($m_{BSA}$, μg) | $m_{bio}/m_{BSA}$ | $\delta D_{SA-SMOW}$ |
|---|---|---|---|
| 0 | | 0 | -88 |
| 0.2 | | $0.04\times10^{-3}$ | -51 |
| 0.5 | 5000 | $0.10\times10^{-3}$ | 22 |
| 1 | | $0.20\times10^{-3}$ | 55 |
| 2 | | $0.40\times10^{-3}$ | 291 |

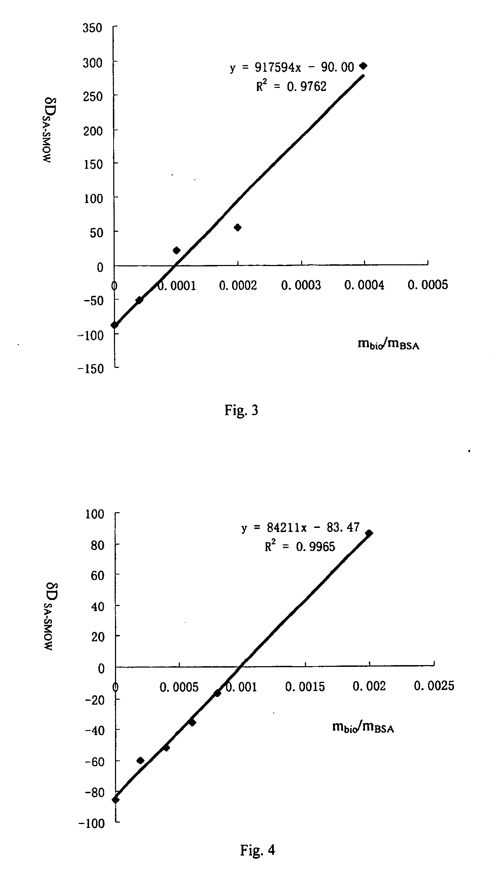

The $\delta D_{SA-SMOW}$ was plotted against $m_{bio}/m_{BSA}$ (see Fig. 3).

It can be known from Fig. 3 that $R^2$ of the straight line was 0.9762, and thus there was a good linear correlation.

The slope of the straight line was $k = \dfrac{500 \cdot C_{bio}^{D}}{R_{SMOW} \cdot C_{BSA}^{H}} = 917594$, and the intercept was - 90.00 = $\delta D_{BSA-SMOW}$, which was consistent with the determined value for BSA, $\delta D_{BSA-SMOW}$=-88.

Calculation of the percent content of deuterium in the deuterated HAV

(a) The amino acid composition of BSA was known from literatures, and the total number of hydrogen atoms was calculated as 4614 and was then divided by 66408.77, the theoretical molecular weight of BSA, and thereby the theoretical percent content of $^1H$ was obtained as $C_{BSA}^{H} = 6.95\%$.

(b) According to the slope of the straight line, the percent content of deuterium in the deuterated HAV was calculated as

$$C_{bio}^D = 917594 \times R_{SMOW} \times C_{BSA}^H \times \frac{1}{500}$$

$$= 917594 \times 155.76 \times 10^{-6} \times 6.95\% \times \frac{1}{500}$$

$$= 1.99\%$$

Example 3

Confirmation of hydrogen/deuterium exchange in the ribonucleic acid (RNA) of deuterated HAV and determination of the percent content of deuterium thereof

The well-washed deuterated HAV or cell control was used to prepare the deuterated RNA, and the method referred to one-step extraction method with guanidine isothiocyanate-phenol. RNA sample was quantified by spectrophotometry, and the $OD_{260}/OD_{280}$ ratio was calculated to characterize the purity of RNA.

BSA was selected as matrix. The RNAs extracted from the deuterated HAV, the non-deuterated HAV and the deuterated cell control were subjected respectively to the following manipulations: the RNA was diluted with PBS to form a diluted sample solution having a desired concentration, 0.1 ml of the diluted sample solution was taken and added into 0.5 ml of PBS solution containing 5 mg BSA. Then, the mixed sample was placed into a lyophil apparatus of Type GEL-1 for lyophilization to such an extent that the water content of the mixed sample was <3% after lyophilization. The resulting samples are represented with RNA-$V_D$, RNA-$V_H$ and RNA-$C_D$, respectively. Adsorbed water in the lyophilized mixed sample and the oxidants CuO (500 mg) and $V_2O_5$ (50 mg) was removed at a temperature of 100°C for 20 minutes, and then the oxidation reaction of the lyophilized mixed sample with the oxidants was carried out by burning at 850°C for 30 minutes, after complete reaction the sample was fully oxidized to carbon dioxide ($CO_2$), water ($H_2O$), nitrogen dioxide ($NO_2$) and so on. The water in the product was separated and transferred into a reaction tube for water sample, and then the water was reacted with zinc at 420°C for 2 hours and thereby converted into hydrogen gas, the $^2H/^1H$ ratio of which was determined with a gas isotope mass spectrometer MAT-251.

The values of $\delta D_{SA\text{-}SMOW}$ of samples RNA-$V_D$ and RNA-$V_H$ were determined. The results showed that the RNA of deuterated HAV has a relatively high deuterium content, and the value of $\delta D_{SA\text{-}SMOW}$ of RNA-$V_D$ increased with the increase in the amount of RNA added (see Table 4). Whereas, the deuterium content of the non-deuterated viral sample RNA-$V_H$ or the deuterated cell control sample RNA-$C_D$ was extremely low, and this indicated that the increase in $\delta D_{SA\text{-}SMOW}$ value of sample RNA-$V_D$ was due to the high $^2H/^1H$ ratio in the added RNA of deuterated HAV, and the increase in $^2H/^1H$ ratio in the RNA of deuterated HAV was due to the exchange of hydrogen by deuterium.

Table 4. The relation between the $\delta D_{SA\text{-}SMOW}$ of sample RNA-$V_D$ and the added amount of deuterated RNA

| Amount of the deuterated RNA ($m_{bio}$, μg) | Amount of the matrix BSA ($m_{BSA}$, μg) | $m_{bio}/m_{BSA}$ | $\delta D_{SA\text{-}SMOW}$ |
|---|---|---|---|
| 0 | | 0 | -85 |
| 1 | | $0.2 \times 10^{-3}$ | -60 |
| 2 | 5000 | $0.4 \times 10^{-3}$ | -52 |
| 3 | | $0.6 \times 10^{-3}$ | -36 |
| 4 | | $0.8 \times 10^{-3}$ | -17 |
| 10 | | $2 \times 10^{-3}$ | 86 |

The $\delta D_{SA\text{-}SMOW}$ was plotted aganist $m_{bio}/m_{BSA}$ (see Fig. 4).

It can be known from Fig. 4 that $R^2$ of the straight line is 0.9965, and thus there was a good linear correlation.

The slope of the straight line was $k = \dfrac{500 \cdot C_{bio}^D}{R_{SMOW} \cdot C_{BSA}^H} = 84211$, and the intercept was -83.474 = $\delta D_{BSA\text{-}SMOW}$, which is consistent with the determined value for BSA, $\delta D_{BSA\text{-}SMOW}$ = -85.

Calculation of the percent content of deuterium in the RNA of deuterated HAV

(a) The amino acid composition of BSA was known from literatures, and the total number of hydrogen atoms was calculated as 4614 and then divided by 66408.77, the theoretical molecular weight of BSA, and thereby the theoretical percent content of $^1$H was obtained as $C_{BSA}^{H} = 6.95\%$.

(b) According to the slope of the straight line, the percent content of deuterium in the RNA of deuterated HAV was calculated as

$$C_{bio}^{D} = 84211 \times R_{SMOW} \times C_{BSA}^{H} \times \frac{1}{500}$$

$$= 84211 \times 155.76 \times 10^{-6} \times 6.95\% \times \frac{1}{500}$$

$$= 0.18\%$$

Example 4

Determination of the percent content of deuterium in the deuterated bovine serum albumin (BSA) sample

The deuteration of BSA was carried out as follows: 500 mg of BSA was diluted in 10 ml of phosphate-buffered saline (PBS) containing 90% heavy water, and thereto the protease inhibitor phenylmethylsulfonyl fluoride (PMSF) was added to a final concentration of 1 mM, and the bacteriostat sodium azide was added to a final concentration of 0.2 mg/ml, and then the resulting mixture was incubated at 20°C for one week. The deuterated BSA was dialyzed against 1000 ml of PBS, and the solution was changed four times in order to completely remove the free heavy water. The deuterated BSA sample obtained after the last dialysis was determined for its protein concentration by spectrophotometry.

BSA was selected as matrix. The deuterated BSA was diluted with PBS to form a diluted sample solution having a desired concentration, 0.1 ml of the diluted sample solution was taken and added into 0.5 ml of PBS solution containing 5 mg BSA. Then, the mixed sample was placed into a lyophil apparatus of Type GEL-1 for lyophilization to such an extent that the water content of the mixed sample was <3% after lyophilization, and thereby the deuterated BSA sample to be tested was obtained. Adsorbed water in the lyophilized mixed sample and the oxidants CuO (500 mg) and $V_2O_5$ (50 mg) was removed at a temperature of 100°C for 20 minutes, and then the oxidation reaction of the lyophilized mixed sample with the oxidants was carried out by burning at 850°C for 30 minutes so that after complete reaction the sample was fully oxidized to carbon dioxide ($CO_2$), water ($H_2O$), nitrogen dioxide ($NO_2$) and so on. The water in the product was separated and transferred into a reaction tube for water sample, and then the water was reacted with zinc at 420°C for 2 hours and thereby converted into hydrogen gas, the $^2$H/$^1$H ratio of which was determined with a gas isotope mass spectrometer MAT-251.

The $\delta D_{SA-SMOW}$ value of the deuterated BSA sample was determined. The results showed that the deuterated BSA had an increased content of deuterium (see Table 5).

Table 5. The relation between the $\delta D_{SA-SMOW}$ of the deuterated BSA sample and the added amount of the deuterated BSA

| Amount of the deuterated BSA ($m_{bio}$, μg) | 0 | 30 | 50 | 70 | 90 |
|---|---|---|---|---|---|
| Amount of the matrix BSA ($m_{BSA}$, μg) | 5000 | | | | |
| $m_{bio}/m_{BSA}$ | 0 | $6 \times 10^{-3}$ | $10 \times 10^{-3}$ | $14 \times 10^{-3}$ | $18 \times 10^{-3}$ |
| $\delta D_{SA-SMOW}$ | -116 | -92 | -70 | -52 | -39 |

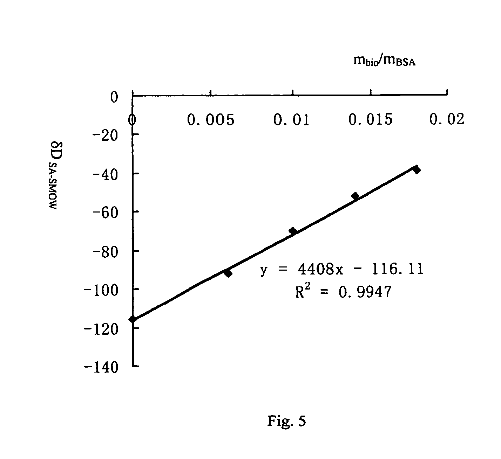

The $\delta D_{SA-SMOW}$ was plotted against $m_{bio}/m_{BSA}$ (see Fig. 5).

It can be known from Fig. 5 that $R^2$ of the straight line was 0.9947, and thus there was a good linear correlation.

The slope of the straight line was $k = \dfrac{500 \cdot C_{bio}^{D}}{R_{SMOW} \cdot C_{BSA}^{H}} = 4408$, and the intercept was -116.11 = $\delta D_{BSA\text{-}SMOW}$, which

was consistent with the determined value for BSA, $\delta D_{BSA\text{-}SMOW} = -116$.

Calculation of the percent content of deuterium in the deuterated BSA

(a) The amino acid composition of BSA was known from literatures, and the total number of hydrogen atoms was calculated as 4614 and then divided by 66408.77, the theoretical molecular weight of BSA, and thereby the theoretical percent content of $^1$H was obtained as $C_{BSA}^{H} = 6.95\%$.

(b) According to the slope of the straight line, the percent content of deuterium in the deuterated BSA was calculated as

$$C_{bio}^{D} = 4408 \times R_{SMOW} \times C_{BSA}^{H} \times \frac{1}{500}$$
$$= 4408 \times 155.76 \times 10^{-6} \times 6.95\% \times \frac{1}{500}$$
$$= 0.01\%$$

## Claims

1. A method for determining the percent content of deuterium in the structure of a biological sample, comprising the following steps:

(1) selecting a known natural protein as matrix, and formulating it with PBS to form a matrix solution having an appropriate concentration;
(2) diluting a biological sample obtained after purification with PBS to various concentrations, subjecting multiple sample solutions that have different concentrations but the same volume respectively to the following manipulations: mixing homogeneously the sample solution with a suitable amount of the matrix solution followed by lyophilization to form a lyophilized mixed sample, sufficiently oxidizing the lyophilized mixed sample with oxidants through burning in order to oxidize the hydrogen in the mixed sample to water, reacting the resulting water after separation with zinc to generate hydrogen gas, and determining the $^2$H/$^1$H ratio of the generated hydrogen gas with a gas isotope mass spectrometer; and
(3) according to a formula, calculating the percent content of deuterium in the biological sample by utilizing the above $^2$H/$^1$H ratios of hydrogen gas determined for the multiple samples, by

(a) plotting the determined $^2$H/$^1$H ratios of the multiple samples (expressed with $\delta D_{SA\text{-}SMOW}$) against the amount of biological sample/amount of matrix protein (expressed with $m_{bio}/m_{MAT}$), and thus obtaining a straight line having a good linear correlation, $\delta D_{SA\text{-}SMOW} = k \cdot \dfrac{m_{bio}}{m_{MAT}} + \delta D_{MAT\text{-}SMOW}$, and educing the slope of the straight line, $k = \dfrac{500 \cdot C_{bio}^{D}}{R_{SMOW} \cdot C_{MAT}^{H}}$;

(b) calculating the theoretical percent content of $^1$H in the matrix protein, $C_{MAT}^{H}$, according to the amino acid composition of the matrix protein;

(c) according to the slope k, calculating the percent content of deuterium in the biological sample as

$$C_{bio}^{D} = k \times R_{SMOW} \times C_{MAT}^{H} \times \frac{1}{500},$$ wherein $R_{SMOW}$ represents the $^2$H/$^1$H ratio of SMOW and its value is $155.76 \times 10^{-6}$,

wherein the said known natural protein is bovine serum albumin (BSA).

2.  The method for determining the percent content of deuterium in the structure of a biological sample according to claim 1, **characterized in that** said biological sample is a microorganism sample or a biomacromolecule sample.

3.  The method for determining the percent content of deuterium in the structure of a biological sample according to claim 2, **characterized in that** said biomacromolecule is a ribonucleic acid (RNA), a protein or a polypeptide.

4.  Use of the method for determining the percent content of deuterium in the structure of a biological sample according to claim 1 in ascertaining the deuterium content of a biological sample having the optimal heat stability or ascertaining the optimal deuteration conditions of a biological sample.

5.  Use of the method for determining the percent content of deuterium in the structure of biological sample according to claim 1 in confirming the close correlation between the stability and heat resistance of a microorganism or a biomacromolecule and the deuterium content thereof.


**Patentansprüche**

1.  Verfahren zur Bestimmung des prozentualen Gehalts an Deuterium in der Struktur einer biologischen Probe, umfassend die folgenden Schritte:

    (1) Auswählen eines bekannten natürlichen Proteins als Matrix, und Formulierung desselben mit PBS, um eine Matrixlösung mit einer entsprechenden Konzentration zu bilden;
    (2) Verdünnen einer nach Reinigung mit PBS erhaltenen biologischen Probe auf verschiedene Konzentrationen, Unterwerfen mehrerer Probenlösungen, die unterschiedliche Konzentrationen, aber jeweils das gleiche Volumen aufweisen, den folgenden Behandlungen: homogenes Mischen der Probelösung mit einer geeigneten Menge der Matrixlösung, gefolgt von einer Lyophilisierung, um eine lyophilisierte Mischprobe zu bilden, ausreichendes Oxidieren der lyophilisierten Mischprobe mit Oxidationsmitteln durch Verbrennen, um den Wasserstoff in der Mischprobe zu Wasser zu oxidieren, Reagierenlassen des resultierenden Wassers nach seiner Abtrennung mit Zink, um Wasserstoffgas zu erzeugen, und Bestimmen des $^2$H/$^1$H-Verhältnisses des erzeugten Wasserstoffgases mit einem Gasisotop-Massenspektrometer; und
    (3) Berechnen des prozentualen Gehalts an Deuterium in der biologischen Probe gemäß einer Formel durch Verwendung der oben genannten, für die mehreren Proben bestimmten $^2$H/$^1$H-Verhältnisse des Wasserstoffgases durch

    (a) Auftragen der bestimmten $^2$H/$^1$H-Verhältnisse der mehreren Proben (ausgedrückt als $\delta D_{SA\text{-}SMOW}$) gegen den Gehalt der biologischen Probe/Gehalt an Matrixprotein (ausgedrückt als $m_{bio}/m_{MAT}$), und **dadurch** Erhalten einer Geraden, $\delta D_{SA\text{-}SMOW} = k \cdot \frac{m_{bio}}{m_{MAT}} + \delta D_{MAT\text{-}SMOW}$ mit einer guten linearen Korrelation und Ableitung der Steigung $k = \frac{500 \cdot C_{bio}^{D}}{R_{SMOW} \cdot C_{MAT}^{H}}$ der Geraden;

    (b) Berechnen des theoretischen prozentualen Gehalts an $^1$H, $C_{MAT}^{H}$, in dem Matrixprotein gemäß der Aminosäurezusammensetzung des Matrixproteins;

    (c) Berechnen des prozentualen Gehalts an Deuterium in der biologischen Probe gemäß der Steigung k als $C_{bio}^{D} = k \times R_{SMOW} \times C_{MAT}^{H} \times \frac{1}{500}$, worin $R_{SMOW}$ das $^2$H/$^1$H-Verhältnis von SMOW repräsentiert und sein Wert $155{,}76 \times 10^{-6}$ beträgt,
    worin das bekannte natürliche Protein Rinderserumalbumin (bovine serum albumin, BSA) ist.

**2.** Verfahren zur Bestimmung des prozentualen Gehalts an Deuterium in der Struktur einer biologischen Probe gemäß Anspruch 1, **dadurch gekennzeichnet, dass** die biologische Probe eine Mikroorganismus-Probe oder eine Biomakromolekül-Probe ist.

**3.** Verfahren zur Bestimmung des prozentualen Gehalts an Deuterium in der Struktur einer biologischen Probe gemäß Anspruch 2, **dadurch gekennzeichnet, dass** das Biomakromolekül eine Ribonukleinsäure (ribonucleic acid, RNA), ein Protein oder ein Polypeptid ist.

**4.** Verwendung des Verfahrens zur Bestimmung des prozentualen Gehalts an Deuterium in der Struktur einer biologischen Probe gemäß Anspruch 1 bei der Bestimmung des Deuteriumgehalts einer biologischen Probe mit einer optimalen Hitzestabilität oder der Bestimmung der optimalen Deuterierungsbedingungen einer biologischen Probe.

**5.** Verwendung des Verfahrens zur Bestimmung des prozentualen Gehalts an Deuterium in der Struktur einer biologischen Probe gemäß Anspruch 1 bei der Bekräftigung der engen Korrelation zwischen der Stabilität und der Hitzebeständigkeit eines Mikroorganismus oder eines Biomakromoleküls und dessen Deuteriumgehalt.


**Revendications**

**1.** Procédé pour déterminer la teneur en pourcentage de deutérium dans la structure d'un échantillon biologique, comprenant les étapes suivantes :

(1) sélectionner une protéine naturelle connue comme matrice, et procéder à sa formulation avec du PBS pour former une solution de matrice ayant une concentration appropriée ;
(2) diluer un échantillon biologique obtenu après purification avec du PBS à différentes concentrations, exposer des multiples solutions d'échantillons qui ont différentes concentrations mais le même volume respectivement aux manipulations suivantes : mélanger de manière homogène la solution d'échantillon avec une quantité appropriée de la solution de matrice suivie par une lyophilisation pour former un échantillon mélangé lyophilisé, oxyder de manière suffisante l'échantillon mélangé lyophilisé avec des oxydants par combustion afin d'oxyder l'hydrogène dans l'échantillon mélangé en eau, faire réagir l'eau résultante après séparation avec du zinc pour générer de l'hydrogène gazeux, et déterminer le rapport $^2$H/$^1$H de l'hydrogène gazeux généré avec un spectromètre de masse isotopique gazeux ; et
(3) selon une formule, calculer la teneur en pourcentage de deutérium dans l'échantillon biologique en utilisant les rapports $^2$H/$^1$H ci-dessus d'hydrogène gazeux déterminés pour les multiples échantillons, en

(a) traçant les rapports $^2$H/$^1$H déterminés des multiples échantillons (exprimés par $\delta D_{SA-SMOW}$) en fonction de la quantité d'échantillons biologiques/quantité de protéines de matrice (exprimée par $m_{bio}/m_{MAT}$), et en obtenant ainsi une ligne droite ayant une bonne corrélation linéaire,

$$\delta D_{SA-SMOW} = k \cdot \frac{m_{bio}}{m_{MAT}} + \delta D_{MAT-SMOW} ,$$ et en extrapolant la pente de la ligne droite,

$$k = \frac{500 \cdot C_{bio}^D}{R_{SMOW} \cdot C_{MAT}^H} ;$$

(b) calculant la teneur en pourcentage théorique de $^1$H dans la protéine de matrice, $C_{MAT}^H$, selon la composition d'acides aminés de la protéine de matrice ;

(c) selon la pente k, calculant la teneur en pourcentage de deutérium dans l'échantillon biologique par

$$C_{bio}^D = k \times R_{SMOW} \times C_{MAT}^H \times \frac{1}{500} ,$$

où $R_{SMOW}$ représente le rapport $^2$H/$^1$H de SMOW et sa valeur est égale à 155,76 x 10$^{-6}$,
où ladite protéine naturelle connue est la sérum-albumine bovin (BSA).

**2.** Procédé pour déterminer la teneur en pourcentage de deutérium dans la structure d'un échantillon biologique selon la revendication 1, **caractérisé en ce que** ledit échantillon biologique est un échantillon de micro-organisme ou un échantillon de biomacromolécule.

3.  Procédé pour déterminer la teneur en pourcentage de deutérium dans la structure d'un échantillon biologique selon la revendication 2, **caractérisé en ce que** ladite biomacromolécule est un acide ribonucléique (ARN), une protéine ou un polypeptide.

4.  Utilisation du procédé pour déterminer la teneur en pourcentage de deutérium dans la structure d'un échantillon biologique selon la revendication 1, pour vérifier la teneur en deutérium d'un échantillon biologique ayant la stabilité thermique optimale ou vérifier les conditions de deutération optimales d'un échantillon biologique.

5.  Utilisation du procédé pour déterminer la teneur en pourcentage de deutérium dans la structure d'un échantillon biologique selon la revendication 1, pour confirmer la corrélation étroite entre la stabilité et la résistance thermique d'un micro-organisme ou d'une biomacromolécule et sa teneur en deutérium.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Mao CS ; Huang CH.** *Sci. Sin.,* 1965, vol. 14 (6), 885-890
- **Wu R ; Georgescu MM ; Delpeyroux F.** *Vaccine,* 1995, vol. 13, 1393-1399
- **Ikizler MR ; Wright PF.** *Vaccine,* 2002, vol. 20
- **Abrams, S. A. ; Wong, W. W.** Book. New York CABI Publishing, 2003
- **Platzner IT ; Habfast K ; Walder AJ. et al.** Book. New York John Wiley & Sons Ltd, 1997

- **Armellin S ; Brenna E ; Fronza G et al.** *Analyst.,* February 2004, vol. 129 (2), 130-3
- **Roger O ; Lavigne R ; Mahmoud M et al.** *J Biol Chem.,* 11 June 2004, vol. 279 (24), 24923-8
- **WONG W W et al.** *AMERICAN JOURNAL OF CLINICAL NUTRITION,* 1987, vol. 45 (5), ISSN 0002-9165, 905-913
- **WONG W W et al.** *JOURNAL OF LIPID RESEARCH,* 1991, vol. 32 (6), 1049-1056
- **MAO J. et al.** *CHINESE SCIENCE BULLETIN,* February 2004, vol. 49 (3), 253-257